(19) Europäisches Patentamt
European Patent Office
Office européen des brevets

(11) Publication number: 0 386 690 A1

(12) # EUROPEAN PATENT APPLICATION

(21) Application number: 90104248.1

(22) Date of filing: 06.03.90

(51) Int. Cl.5: G01N 33/542, G01N 33/58, G01N 33/553, //G01N33/569

(30) Priority: 08.03.89 US 320738

(43) Date of publication of application: 12.09.90 Bulletin 90/37

(84) Designated Contracting States: AT BE CH DE DK ES FR GB GR IT LI LU NL SE

(71) Applicant: Becton Dickinson and Company
One Becton Drive
Franklin Lakes New Jersey 07417-1880(US)

(72) Inventor: Vonk, Glenn P.
2717 Piney Grove-Wilbon Road
Fuquay-Varina, North Carolina(US)
Inventor: Schram, James L.
102 Dwelling Place
Knightdale, North Carolina(US)

(74) Representative: von Kreisler, Alek, Dipl.-Chem. et al
Patentanwälte Von Kreisler-Selting-Werner
Deichmannhaus am Hauptbahnhof
D-5000 Köln 1(DE)

(54) **Signal enhancement in magnetic immunoassay by inhibition of enzymatic catalysis.**

(57) A method for enzyme immunoassay for a ligand suspected to be present in a liquid sample includes signal amplification by use of at least two enzymes and a blocked inhibitor for one of the enzymes. Ligand present in the liquid binds to an antiligand conjugated to a magnetic particle and an enzyme-labeled tracer. After binding, the particles are separated magnetically and the enzyme in the tracer removes the blocking group from the blocked inhibitor. The inhibitor inhibits a second enzyme which catalyzes the conversion of a substrate to a product. The presence or absence of the ligand in the liquid is indicated by a signal, such as a color change or a rate of color change. The invention includes a kit of materials useful for performing the method of the invention.

EP 0 386 690 A1

# SIGNAL ENHANCEMENT IN MAGNETIC IMMUNOASSAY BY INHIBITION OF ENZYMATIC CATALYSIS

## 1. Field of the Invention.

This invention relates to magnetic immunoassay of an analyte and materials used therein, and more particularly relates to a method and materials for immunoassay in which enhancement of a detectable signal is achieved by inhibition of enzymatic catalysis of an indicator reaction.

## 2. Background of the Invention.

Assay systems which are both rapid and sensitive have been developed to determine the concentration of a substance in a fluid. Immunoassays depend on the binding of an antigen or hapten to a specific antibody and have been particularly useful because they give high levels of specificity and sensitivity. These assays generally employ one of the above reagents in labeled form, the labeled reagent often being referred to as the tracer. Immunoassay procedures may be carried out in solution or on a solid support and may be either heterogeneous, requiring a separation of bound tracer from free (unbound) tracer or homogeneous in which a separation step is not required.

A variety of methods has been used to separate bound tracer from free tracer. In one procedure, magnetic particles having antibodies conjugated thereto are used as the solid phase, and the separation step is accomplished with a magnet which pulls the particles, after the binding reaction, to the walls of the assay vessel so that unbound tracer can be decanted off. Exemplary of immunoassay procedures using magnetic particles are those disclosed in U.S. Patent Np. 4,777,145 to Luotola et al. and 4,745,077 to Holian et al.

Immunoassay labels are generally radioisotopes, fluorochromes or enzymes. In conventional enzyme immunoassay (EIA), an enzyme is covalently conjugated with one component of a specifically binding antigen-antibody pair, and the resulting enzyme conjugate is reacted with a substrate to produce a signal which is detected and measured. When the signal is a color change, detection with the naked eye is limited because the average individual can detect the presence of chromophores only down to about $10^{-5}$ or $10^{-6}$ M.

EIA sensitivity can often be increased by spectrophotometric techniques; however, these procedures require expensive equipment. In another approach, the sensitivity may be increased by various amplification methods. Single enzyme amplification methods have been disclosed in which ligands present at concentrations of $10^{-6}$ to $10^{-10}$ M have been detected. These methods however, have been generally unsatisfactory at ligand concentrations below $10^{-11}$ M. In cascade amplification procedures, the number of detectable (generally colored) molecules is increased by use of two or more enzymes or enzyme derivatives. U.S. Patent No. 4,463,090 to Harris discloses a cascade amplification immunoassay in which a large molecule activator, such as an enzyme or a proenzyme coupled to a ligand, activates a second enzyme which reacts with a substrate to produce a detectable signal or in turn activates a third enzyme.

U.S. Patent No. 4,446,231 to Self discloses a cycling amplification enzyme immunoassay which includes primary and secondary enzyme systems and a modulator for the second enzyme system. The primary system includes a first enzyme coupled to a ligand. In a first embodiment of the Self invention, the first enzyme system acts on a modulator precursor to liberate a modulator. The modulator is a cofactor of the secondary enzyme which activates the second enzyme system to catalyze the reaction of a substrate to a detectable product. During the reaction, the modulator is converted to an inactive form, and cycling is accomplished by a third enzyme which reactivates the modulator. In a second embodiment the modulator is an inhibitor of the secondary system, and is removed by the primary enzyme system whereby the secondary system is activated to act on the substrate and thereby produce the detectable product.

Boguslaski et al., U.S. Patent No. 4,492,751 teaches a cycling system in which an enzyme substrate or coenzyme is conjugated to one member of the specifically binding pair.

A variety of molecules has been shown to cause specific inactivation of a target enzyme. A subset of inhibitors, termed mechanism-based inhibitors, are substrates for enzymes which react with an enzyme to form a covalent bond. Mechanism-based inhibitors have been reviewed by Walsh (Tetrahedron 38, 871 (1982). Another subset of inhibitors includes molecules which act as stable transition-state analogs. Gelb et al. have disclosed some fluoroketones as transition-state inhibitors of hydrolytic enzymes in Biochemistry 24, 1813 (1985).

## SUMMARY OF THE INVENTION

One aspect of the present invention is a method for detection of a ligand in a liquid sample, hereinafter referred to as the unknown sample. The unknown sample suspected of containing the ligand is combined with a specific antiligand conjugated to a magnetic particle and a tracer for the ligand. The tracer includes a first hydrolase conjugated to either the ligand or to a second antiligand, also specific for the ligand. Conditions conducive to binding between ligand, antiligand and tracer are provided to give a bound phase on the particle. After separation of the particle having the bound phase thereon from the fluid by pulling the particles to the wall of the assay vessel with a magnet and aspirating or decanting the liquid, the bound phase is contacted in a liquid with a blocked inhibitor and a second hydrolase. The first hydrolase removes the blocking group to provide an inhibitor for the second hydrolase. A substrate for the second hydrolase is then added. The substrate is converted by the second hydrolase to a product which provides a detectable signal, the conversion of the substrate to the product by the second hydrolase being inhibited by the inhibitor.

The ligand may be an antigen, a hapten or an antibody. The preferred ligand is an antigen, most preferably a viral antigen. The method may be carried out by a competitive immunoassay technique, in which case the tracer is the ligand conjugated to the first hydrolase. Preferably, a sandwich immunoassay technique may be used, in which case the tracer is a second antiligand conjugated to the first hydrolase.

The preferred blocked inhibitor of the present invention is a blocked inhibitor which is converted by the first hydrolase component of the tracer to an inhibitor. The preferred substrate is a chromogen which is convertible by the second hydrolase to a product of a different color. Most preferably the chromogen is colorless and is converted to a colored product by the second hydrolase, the conversion of chromogen to product being inhibited by the inhibitor.

In the most preferred assay format, an antibody may be conjugated to the magnetic particle and contacted under binding conditions with a viral antigen and a second antibody, specific for the antigen, labeled with alkaline phosphatase. After binding and separation, the particle having an antibody:antigen:alkaline phosphatase-labeled antibody sandwich affixed thereto is contacted with the blocked inhibitor and an esterase in a liquid. If antigen is present in the fluid, alkaline phosphatase captured on the particle removes the blocking group to give the inhibitor. A colorless chromogen is added. Inhibitor generated in the liquid inhibits the esterase from converting the chromogen to the colored product. Thus the development of color indicates the absence of antigen in the sample and failure of color development indicates the presence of antigen in the sample.

Another aspect of the invention includes a kit of materials for performing the method of the invention substantially as described above.

In any EIA system which includes unblocking of an enzyme inhibitor, severe constraints are placed on both the inhibitor and the blocked inhibitor if the assay is to achieve maximum sensitivity. In accordance with the present invention, the blocked inhibitor is an excellent substrate for the first (unblocking) hydrolase, but is essentially unreactive toward the second (color forming) hydrolase. Likewise, the unblocked inhibitor is a potent inhibitor of the second hydrolase, but has essentially no effect on the first hydrolase. Because the reactions of the blocked inhibitor and inhibitor with the first and second hydrolases respectively are highly selective, "short circuits" characteristic of prior art cycling EIA systems due to cross reactivities are substantially eliminated.

Thus, the invention combines a versatile method for assay for ligands present in very low concentrations in a fluid with the simple and efficient separation technique of magnetism. The method makes possible naked eye detection and measurement of an assay signal even though the ligand is present in concentrations as low as $10^{-12}$M and greatly extends the range of ligands which can be detected or determined without expensive or cumbersome equipment. Further, the assay sensitivity, i.e., time required to detect the presence or absence of the ligand, may be reduced by up to 100 fold compared to conventional E1A. Significant savings in cost and space are thereby achieved, enabling assays in accordance with the invention to be carried out in small clinical laboratories or even in a physician's office.

## DETAILED DESCRIPTION OF THE PREFERRED EMBODIMENTS

While this invention is satisfied by embodiments in many different forms, there is described in detail preferred embodiments of the invention, with the understanding that the present disclosure is to be

considered as exemplary of the principles of the invention and is not intended to limit the invention to the embodiments described. The scope of the invention will be measured by the appended claims and their equivalents.

In accordance with the method of the invention, a substance, hereinafter referred to as the ligand, present in the unknown sample, may be detected visually, i.e., by naked eye observation, even when present in very low concentrations. The method includes at least two amplification stages. A first amplification stage is enzymatic unblocking of an enzyme inhibitor. A second amplification stage is enzymatic catalysis of an indicator reaction. These amplification steps take place sequentially to provide signal amplification of $10^6$ fold or higher whereby a ligand present in the sample at a level of $10^{-12}$M or lower may be detected with the naked eye. If additional amplification is desired, a first enzyme may be provided which initiates a cascade of sequential reactions involving a plurality of enzymes, wherein any one or all of the reactions may provide further signal amplification.

An immunological reaction is used in the method of the invention for detection of the ligand in the unknown sample. By the term "immunological reaction," as used herein, is meant a specific binding reaction of an antigen and an antibody, a hapten and an antibody, or any appropriate analogue of an antigen, an antibody, or a hapten which also binds specifically.

The immunological reaction may be carried out in any suitable liquid. For example, the liquid may be a body fluid suspected of containing the ligand, such as serum, urine, cerebrospinal fluid, pleural fluid or the like. Alternatively, the liquid may be water, saline or any appropriate buffer, or a mixture of body fluids and other liquids to which has been added a sample suspected of containing ligand.

The preferred method for sandwich assay of the invention will first be described with reference to the assay flow sheet below to provide a general understanding of the assay components and their interaction, after which each component will be discussed in detail.

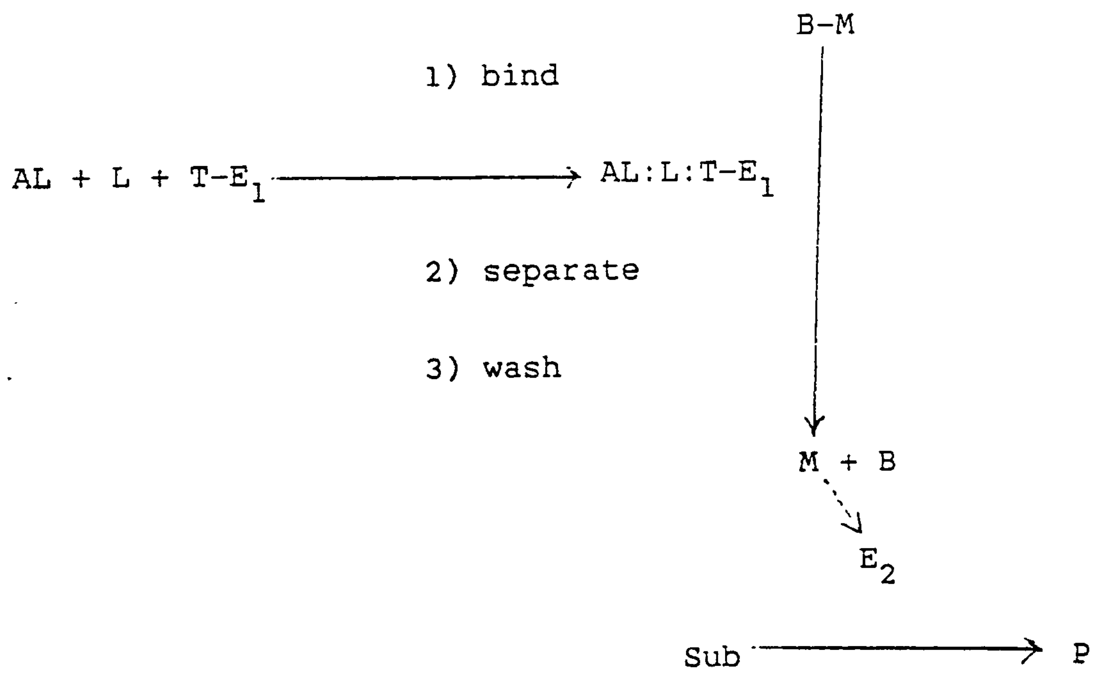


In the above flow sheet, the following definitions apply, wherein a colon indicates an immunological binding, a hyphen indicates a chemical bond or a physical attachment, such as absorption, a solid arrow indicates a chemical conversation and a dotted arrow indicates modulation of a reaction or an assay component.

AL - antiligand
L - ligand
T - tracer
$E_1$ - first enzyme
$E_2$ - second enzyme
B-M - blocked modulator
M - modulator for enzyme
B - blocking group for modulator
Sub - substrate

P - product

It is seen from the flow sheet that conditions conducive to binding of ligand to antiligand and tracer are provided, followed by a separation step and appropriate wash steps. A second enzyme and a blocked modulator consisting of a modulator (activator or inhibitor) and a blocking group are added, and the blocking group is removed by the first enzyme component of the tracer. A substrate for the second enzyme is added, and the conversion of the substrate to a detectable product by the second enzyme is modulated by the modulator liberated from the blocked modulator by the first enzyme. The level of product is either directly or inversely proportional to the level of modulator, and thus to the ligand, depending on whether the modulator is an activator or inhibitor respectively. The actual signal measured may be a color associated with the indicator reaction, as, for example, the color of the product or rate of formation thereof, or the color of the substrate or the rate of disappearance thereof.

The separation step of the present invention is performed by magnetism wherein one or more assay components may be attached to the surface of a magnetic particle. A variety of particles, variously referred to as magnetic or superparamagnetic particles are well-known in the art and have been used in various immunoassay procedures. Many are commercially available, and the invention contemplates use of any particle to which the antiligand can be attached and which can be magnetically pulled to the walls of an assay vessel by an externally applied magnet. Representative suppliers of magnetic particles are Advanced Magnetics, Cambridge, Massachussetts, DuPont, Wilmington, Delaware; Molecular Biosystems Inc., San Diego, California; and Immunicon, Huntingdon Valley, Pennsylvania.

Conjugation of the antiligand to the magnetic particle may be performed by any of the well-known and well-described procedures in the art. In general, those procedures involve covalent linking of a functional group on the particle as supplied by the manufacturer to a functional group, such as a hydroxyl, mercapto, amino or carboxyl group, on the antiligand. Linking reagents such as carbodiimides, tosylates and glutaraldehyde are often used for this purpose. Representative procedures are described by Pourfarzaneh et al. (Methods of Biochemical Analysis 28, 267, 1982); Ligand Quarterly 5, 41 (1982)).

Turning now to a detailed description of the assay components, the ligand may be from any source, and may be an antigen, an antibody or a hapten. For example, the ligand may be an antigen present in a body fluid, or it may be isolated from a body fluid and subsequently introduced into a different liquid, such as buffer. In other cases, the ligand may be from a source other than a body fluid, as, for example, a culture of microorganisms or a cellular extract thereof. Preferred ligands are antigens, most preferably viral antigens present in a body fluid, such as Herpes simplex virus (HSV), Adenovirus, Influenza A virus, Parainfluenza 3 virus and RSV.

The antiligand on the surface of the particle may be an antigen or an antibody, either monoclonal or polyclonal, or it may be any appropriate analogue thereof which reacts specifically with the ligand. In addition, the antiligand may be an antibody complex consisting of a plurality of bound antibodies, as, for example, a second antibody bound specifically to a first antibody. Alternatively, the ligand may bind to several different antiligands, for example, an ensemble of polyclonal antibodies or a mixture of several monoclonal antibody molecules which bind simultaneously to different surface areas of the ligand. Generally, the second antibody is raised against the first antibody in a different species. The plurality of bound antibodies in the complex may contain from about two to ten or more antibodies.

In the sandwich assay of the invention, it is preferred to use excess antiligand having sufficient binding sites to bind essentially all of the ligand.

The tracer comprises two components, the first enzyme conjugated to the ligand (competitive assay as described later) or, in the preferred sandwich assay, to a second antiligand. The enzyme may be conjugated to the ligand or second antiligand in any suitable way, preferably by a covalent linkage, prior to the immunological reaction. Covalent conjugation of enzymes to ligands or antiligands is conventional and well known to those skilled in the art.

Any enzyme which can remove a blocking group from a blocked modulator may be used as the first enzyme. Suitable first enzymes are generally hydrolases, such as phosphatases, peptidases, esterases, glycosidases and the like. Exemplary of, but not limited to, suitable first enzymes are trypsin, thrombin, mammalian liver esterase, acetylcholinesterase, β-galactosidase, or most preferably, alkaline phosphatase.

The liquid containing the particles, the ligand and the tracer may be incubated, if necessary, to induce binding. Incubation may be carried out at any temperature and for any length of time suitable to facilitate binding, preferably from about 20° to 40° for about 1 minute to 4 hours. Particles and bound ligand and tracer are hereinafter referred to as the bound fraction and ligand and tracer which do not bind are hereinafter referred to as the free fraction. The assay may, but need not, be carried out in such a way that equilibrium is established between the bound and free fractions.

After binding, the bound fraction may be separated from the free fraction in the liquid phase of the

assay mixture by pulling the particles to the wall of the assay vessel with an externally applied magnet. The fluid may be decanted or aspirated off, the particles washed to remove interfering materials, separated from the wash liquid in the same manner and suspended in a second assay liquid phase such as water, saline or buffer. The blocked modulator may then be added, and the pH is adjusted to any level, preferably 6-8, which does not cause non-enzymatic removal of the blocking group, as described below.

The blocked modulator may be any material which may be converted by the first enzyme to a modulator of the second enzyme. The preferred blocked modulator has two components, the modulator and the blocking group. The most preferred blocked modulator is a blocked inhibitor which is unreactive toward the second enzyme until its blocking group is removed by the first enzyme and the inhibitor is liberated into the assay medium. Thus, the choice of the components of the blocked inhibitor depends on the first and second enzymes to be used. The blocking group should be one which can be covalently conjugated to the inhibitor by a bond which can be cleaved substantially selectively by the first enzyme, and the inhibitor component should inhibit the activity of the second enzyme while having substantially no effect on the first enzyme. Thus, the nature of the second enzyme and its substrate will be discussed prior to further description of the blocked inhibitor and the inhibitor.

In the assay of the invention, the second enzyme is generally a hydrolase which converts the substrate to the product. Suitable hydrolases are, for example, phosphatases, peptidases such as trypsin, chymotrypsin and pepsin, or preferably esterases such as acetyl cholinesterase (AChE) and butyl cholinesterase. The most preferred second enzyme is a carboxyesterase, such as rabbit liver esterase (RLE). When the second enzyme is an esterase, the assay may be configured by conjugating the esterase, in addition to the antiligand, to the magnetic particle.

The substrate may be any substance containing a group which can be cleaved by the second enzyme to provide a product detectable by a signal associated with color. Thus, in one embodiment of the invention, the signal detected is the development or disappearance of a color, or a change from one color to another. In another embodiment of the invention, the signal may be a change in the rate at which the substrate is converted to the product, for example, the color of a substrate may be observed to remain unchanged for a specified length of time. Thus, measurements of the signal may be made under either kinetic or thermodynamic conditions. Kinetic measurements determine the rate of change which occurs over a period of time, and are generally carried out by making a series of measurements at various times after combining the assay reagents. Thermodynamic measurements determine the extent of change which has occurred when equilibrium has been reached between the substrate and the product of the indicator reaction. Measurements may be made either instrumentally or, preferably, with the naked eye.

It is preferred that the substrate be colorless until cleaved by the second enzyme to give a colored product. Suitable substrates are esters of nitrophenols, such as ortho and para nitrophenyl acetates or butyrates. These substrates are colorless until cleavage of the acetyl or butyryl groups by carboxyesterase occurs to give colored nitrophenols. Preferred substrates are esters of indoxyl such as indophenyl butyrate, indophenyl acetate and most preferably 4-[(3,5-dichloro-4-butyryl-oxyphenyl)imino]-2,5-cyclohexadien-1-one, (3′,5′-dichloroindophenyl butyrate) hereinafter referred to as DCIPB.

It is evident that the method of the invention may also be used in a fluorescence immunoassay. In this embodiment of the invention, the second enzyme may convert a nonfluorogenic substrate to a fluorogenic product wherein the signal measured is modulation of fluorescence. For this embodiment of the invention, it is preferred that the modulator be an inhibitor and the second enzyme be an esterase.

As mentioned above, the first enzyme component of the tracer cleaves the blocking group from the blocked inhibitor to provide the inhibitor of the second enzyme. Suitable inhibitors and blocked inhibitors for the present invention are described fully in copending application serial number 932,951 of common assignee, and that portion of the copending application which discloses the preparation and use of blocked inhibitors and inhibitors in a dual enzyme EIA is hereby incorporated by reference.

In the most preferred embodiment of the invention, the tracer is a second antiligand having alkaline phosphatase covalently conjugated thereto. After separation and washing of the particles and resuspension thereof in a suitable assay liquid, RLE or AChE (second enzyme) and a blocked fluoroketone inhibitor are added. If antigen is present in the unknown fluid, it and the tracer are captured on the particle, and the alkaline phosphatase causes cleavage of the blocking group to give free fluoroketone inhibitor. The assay is completed by addition of DCIPB.

If the inhibitor formed due to the presence of antigen in the unknown fluid, the activity of the esterase is inhibited, and colorless DCIPB is not converted to colored dichloroindophenol. If no antigen is present in the unknown fluid, no alkaline phosphatase is captured on the particle, no inhibitor is formed and the colored product therefore forms because the esterase is not inhibited.

In this most preferred embodiment of the invention using $1X10^{-12}M$ alkaline phosphatase and $3X10^{-9}M$

RLE as first and second enzymes respectively, the presence or absence of an antigen in the unknown fluid down to a level of $1X10^{-12}M$ can be detected in about seven minutes. If antigen is present in the unknown fluid, no color detectable with the naked eye (ca $1X10^{-5}M$ of product VIII) develops in this time. If no antigen is present, color does develop. In contrast, by conventional immunoassay using first enzyme (alkaline phosphatase) alone, about 104 minutes are required for the enzyme to cleave sufficient phosphorylated substrate to provide detectable color. Thus, the invention represents a 15 fold increase in assay sensitivity using these reagents at these levels.

As mentioned earlier, another embodiment of the invention is a competitive assay wherein the tracer is a predetermined quantity of the ligand conjugated to the first enzyme. In a competitive assay, the quantity of antiligand conjugated to the particles is insufficient to bind all of the ligand and tracer present in the assay liquid so that ligand and tracer compete for the limited number of antiligand binding sites. Thus, in a competitive assay, the quantities of ligand and tracer which bind to the antiligand (bound fraction) are inversely proportioned to their concentrations in the assay liquid.

If additional signal amplification is desired, a multistage cascade amplification assay may be carried out wherein a plurality of reagents in the assay medium react sequentially leading ultimately to modulator unblocking. In describing this embodiment of the invention, it is convenient to consider the first enzyme described above as a primary enzyme which enzymatically converts a reagent in the assay medium to a secondary enzyme which unblocks the modulator for the above-described second enzyme. Further, the secondary enzyme, or any subsequent enzyme, may react with additional reagents to provide additional enzymes which may continue the cascade of enzymatic reactions until the modulator is unblocked. By proper selection of reagents to be added to the assay medium, any desired number of amplification stages may be carried out.

Amplification occurs in any embodiment of the invention heretofore described because the first enzyme, or any subsequently formed enzyme, and the second enzyme act as true catalysts wherein a single enzyme molecule may act on an essentially unlimited number of blocked modulator or substrate molecules respectively without being consumed. Thus, in theory, one molecule of each enzyme would be sufficient to perform the method of the invention. In practice, determination of the amounts of the enzymes to be added and the number of amplification stages to be used depend on the level of amplification desired and are well within the purview of one of ordinary skill in the art.

It is evident that an almost unlimited number of competitive and sandwich assay configurations which fall within the scope of the invention can be envisioned. Further, the invention provides assay configurations which are suitable for either detection of the ligand or determination of ligand concentration. Ligand concentration may be determined by comparing the magnitude of the signal generated with the unknown sample with the magnitude of the signal measured upon assay of a range of known quantities of the ligand assayed under essentially identical conditions. When the method of the invention is to be used for determination of ligand concentration, it is advantageous to read signal intensity by an appropriate instrument, such as a spectrophotometer, for example, a Beckman DU7 Spectrophotometer, Beckman Instruments, Inc., Irvine, California.

In another embodiment of the invention, the second enzyme may be conjugated to the particle in close proximity to the conjugated antiligand. After the immunological reaction by which the ligand and tracer are captured on the particle, the particle is separated from the liquid phase, washed, and the blocked modulator is added. The first enzyme portion of the tracer reacts with and liberates the modulator for the second enzyme on the particle. The substrate for the second enzyme may then be added and the assay completed as previously described.

Another aspect of the invention is a reagent kit or package of materials for performing an assay for a ligand in accordance with the method of the invention. The kit may include one or more antiligands, a first of which is conjugated to a magnetic particle, a first enzyme which may be conjugated to a second of the antiligands or to the ligand, a second enzyme, and a blocked modulator for the second enzyme. The kit may also include standards for the ligand, as, for example, one or more ligand samples of known concentration, or it may include other reagents, enzyme substrates, or other labeled or unlabeled specific ligands, antiligands or complexes thereof useful in carrying out the assay. It may include solutions, such as saline or buffers. The components of the kit may be supplied in separate containers, as, for example, vials, or two or more of the components may be combined in a single container.

The following examples are provided to further describe the invention but are in no way to be considered as limitative of the invention.

## EXAMPLE I

Preparation of Magnetic Particle - Antibody Conjugate

Magnetic particles having pendant primary amino groups (Advanced Magnetics, Cambridge, Massachussetts) were washed five times with 0.1 M sodium bicarbonate pH 8.0 - 8.3. A samarium cobalt magnet was used to separate the particles from solution after each wash. The supernatant was decanted and a solution of the heterobifunctional crosslinker sulfoSMCC (Pierce, Rockford, Illinois, 50 mg, 120 µmol) in 3 mL of bicarbonate buffer was added. This reagent reacts with primary amines via an N-hydoxysuccinimide ester to install a flexible spacer chain and maleimide for conjugation with thiol containing proteins. The mixture was gently mixed for three hours and the particles washed with protein coupling buffer (50 mM tris, 50 mM sodium acetate, 50 mM sodium chloride, 1 mM EDTA, pH 8.0). Antibodies were reduced with dithiothreitol in reduction buffer (50 mM sodium acetate, 50 mM sodium phosphate (dibasic), 100 mM sodium chloride 5 mM EDTA, pH 6.5) and purified over Sephadex G-25 eluting with protein coupling buffer. Approximately 9 mg of reduced antibody was then added to the particles and the suspension mixed gently overnight. Conjugation efficiency was estimated using protein assay (BCA, Pierce, Rockford, Illinois) of the supernants and was about 90%. The particles were washed with viral extraction reagent (250 mM tris, 150 mM NaCl, 10 mM EDTA, 0.75% sodium glycodeoxycholate, pH 8.5) to remove adsorbed antibody and stored at 4°C in the same buffer containing 0.2% sodium azide. The particles are stable for at least one month under these conditions.

EXAMPLE II

Preparation of AP - Antibody Conjugate (Tracer)

Alkaline phosphatase (Boeringer Mannheim) was dialyzed overnight in potassium phosphate buffer (KPi) (50 mM, pH 7.5). The enzyme (3 mg/mL) was then derivatized with 4-(p-maleimidophenyl) butyrate (SMPB, Sigma) dissolved in DMF (20 mM) at 325 µM final concentration. After thirty minutes at ambient temperature, the reaction was terminated by separation on a Sephadex G-25 column (Pharmacia) using 50 mM. KPi for elution. Care was taken so that no carry over of SMPB occurred. The derivatized enzyme was concentrated with an ultrafiltration cell (Amicon, YM-30 membrane) to 10 mg/mL. One to two moles of SMPB were incorporated per mole of enzyme. Monoclonal anti RSV-48 antibodies (ca. 10 mg/mL) were reduced with dithiothreitol (20 mM) for one hour in antibody reduction buffer (50 mM sodium acetate, 100 mM sodium chloride, 50 mM sodium phosphate 5 mM ethylenediaminetetraacetic acid, pH 6.5) and purified over Sephadex G-25 as described above eluting with 50 mM KPi. The reduced antibody was immediately mixed with SMPB-alkaline phosphatase to give final concentrations of 1.6 and 3.2 mg/mL respectively. The solution was allowed to stand two hours at ambient temperature, then overnight at 4°C. The reaction was then quenched with 2-mercaptoethanol (0.5 mM) and purified on a Sephacryl S-300 (Pharmacia) column previously equilibrated with 50 mM tris, 100 mM sodium chloride, 1 mM $MgCl_2$, pH 7.5. The conjugate was then concentrated to ca. 0.5 mg/mL, and stored in the presence of sodium azide (0.1%) at -20°C.

EXAMPLE III

Preparation of 4-[(3,5-dichloro-4- butyryloxyphenyl)imino]-2,5-cyclohexadien-1-one (DCIPB)

A 1L round bottom flask was charged with 750 mL of acetonitrile and 30.0 g (0.103 mol) of 2,6-dichloroindophenol (sodium salt, hydrate). Butyric anhydride (25 mL, 0.153 mol) was combined with 10 mL of pyridine (0.124 mol) and 10 mL of acetonitrile in an addition funnel. This mixture was added dropwise to

the well-stirred reaction mixture over a period of one hour. Stirring was continued for an additional 16 hours at ambient temperature, at which time the reaction mixture was concentrated by rotary evaporation under reduced pressure. The resulting syrup was diluted with 500 mL of ethyl acetate and washed with 250 mL of 1 N hydrochloric acid, 200 mL of saturated sodium bicarbonate, and dried over anhydrous magnesium sulfate. The crude product was concentrated to a syrup by rotary evaporation. Two isomers were present in the crude material because reaction with butyric anhydride can occur at either of the two reactive phenolate centers. These isomers were separated by flash chromatography on silica in ethyl acetate/hexane (15/85). Purity of the fractions was assessed by TLC in ethyl acetate/hexane (20/80). The desired compound has an Rf of 0.32, while the other isomer runs at 0.38. Fractions contaminated with the undesired isomer were rechromatographed. Pooled product was obtained in a 64% yield (22.2 g). NMR(CDCl3): 1.09(t,3H), 1.86-(m,2H), 2.67(t,2H), 6.66(m,2H), 6.90(s,2H), 7.07(d,1H), 7.25(d,1H).

EXAMPLE IV

Assay for RSV

All operations were performed at ambient temperature. A magnetic separation rack was obtained from Ciba-Corning Diagnostics, Medfield, Massachussetts. A suspension of the antibody-conjugated particles from Example I was pipeted into a glass test tube, the particles separated, and the supernatant aspirated. The analyte solution (100 uL) in viral extraction reagent was added, vortexed, and allowed to stand one minute. To this was added the monoclonal antibody-AP conjugate of Example II in 1 mL of assay buffer (100 mM tris, 150 mM sodium chloride, 0.02% sodium azide, 0.1% casein (Gallard-Schlesinger, Carle Place, New York, hammersten grade), pH 7.5). The mixture was vortexed, and allowed to stand five minutes. The particles were pulled to the side of the tube with a magnet and the supernatant aspirated. Assay buffer (1 mL) was added and aspirated. The particles were then washed twice with 2 mL of assay buffer (vortexing each time) and the final wash aspirated. Rabbit liver esterase (0.8 µg/mL) and blocked fluoroketone inhibitor (0.15 mM) solution in DEOA buffer (50 mM diethanolamine, 50 µM $MgCl_2$ pH 9.0, 500 µL) were added to all tubes and a control tube. The tubes were vortexed and allowed to stand for twenty minutes. DCIPB, (Example III) 0.35 mM in substrate buffer (50 mM tris HCl, 20% methanol, pH 7.5, 250 µL) was added to develop color. After five minutes stop buffer (50 mM tris, 1 mM 2,2,2-trifluoroacetophenone, 0.1% methanol, pH 7.5, 50 µL) was added to stop the esterase reaction. The total procedure requires ca. 40 minutes. Absorbance was measured at 620 nm and was stable for at least one hour.

EXAMPLE V

Comparison of Dual Enzyme Assay of the Invention with Commercial Assay

Fifty clinical samples known to be positive for RSV were pooled, and the pooled specimen assayed by the method of the invention and by a commercial enzyme linked immunosorbent assay (RSV-ELISA, Ortho Diagnostic Corp., Raritan, New Jersey). By the assay of the invention in accordance with Example IV, the pooled specimen still tested positive after a 1:1500 dilution of the pool with viral extraction reagent. In contrast, the pooled specimen was negative when diluted only 1:250 and tested according to the manufacturer's directions for the Ortho RSV-ELISA. Thus, the assay of the invention was shown to be at least six times as sensitive as the commercial assay.

EXAMPLE VI

Co-conjugation of Anti-RSV 97 Antibodies and RLE

RLE was reduced with Dithiothreitol using the same procedure described in Example I for the reduction of antibodies. In this fashion, RLE was conjugated to magnetic particles with retention of much of its activity. The efficacy of this approach was tested by reducing both monoclonal anti-RSV 97 and RLE separately, then combining them in various proportions and adding them to magnetic particles which possessed a maleimide (sulfo-SMCC). This gave particles having both RLE and anti-RSV-97 attached. Particles prepared in this fashion were used as previously described to form a sandwich with RSV antigen and monoclonal anti-RSV 48 - alkaline phosphatase conjugate. The particles were separated, washed, and only blocked inhibitor added (no RLE) in 50 mM diethanolamine, pH 9.0). Following this, DCIPB (RLE substrate) was added. Color formation was inversely proportional to the amount of RSV antigen added. Comparison of this assay with the assay of the invention using particles having only anti-RSV 97 showed no significant differences. The advantage of this system is that a reagent addition (RLE) is eliminated by placing that reagent on the particles.

Thus, the invention combines a magnetic separation configuration with a dual enzyme assay format including enzymatic deblocking by one enzyme of a blocked inhibitor of a second enzyme. The method provides signal amplification by $10^6$ fold or more, enabling naked eye detection of the signal in a time up to 100 fold less than conventional EIA.

## Claims

1. A method for the detection of a ligand to a liquid comprising:

(a) combining in an assay vessel a first liquid suspected of containing a ligand with an antiligand conjugated to a magnetic particle and a tracer, said tracer comprising a first hydrolase, whereby said antiligand binds to said ligand and said tracer binds to one of said antiligand and said ligand to give a bound phase on said particle;

(b) separating said particle from said first liquid;

(c) contacting said particle with a second liquid containing a second hydrolase and a blocked fluoroketone whereby said first hydrolase converts said blocked fluoroketone to a fluoroketone and releases said fluoroketone into said second liquid;

(d) adding to said second liquid a substrate for said second hydrolase whereby conversion of said substrate to a produce by said second hydrolast is inhibited by said fluoroketone; and

(e) detecting said ligand by a signal associated with said conversion.

2. The method in accordance with Claim 1 wherein said tracer binds to said antiligand and further comprises said ligand having said first hydrolast conjugated thereto.

3. The method in accordance with Claim 1 wherein said tracer binds to said ligand and further comprises a second antiligand having said first hydrolase conjugated thereto.

4. The method in accordance with Claim 1 wherein the step of detecting by a signal includes detecting a color associated with said product.

5. A method for the detection of a ligand in a liquid comprising:

(a) combining in an assay vessel a first liquid suspected of containing a ligand with an antiligand conjugated to a magnetic particle and a tracer, said tracer comprising a first hydrolase, whereby said antiligand binds to said ligand and said tracer binds to one of said ligand and said antiligand to give a bound phase on said particle;

(b) separating said particle from said first liquid;

(c) contacting said particle in a second liquid with a second hydrolase and a blocked inhibitor whereby said first hydrolase converts said blocked inhibitor to an inhibitor and thereby releases said inhibitor into said second liquid;

(d) adding to said second liquid a substrate for said second hydrolase whereby a conversion of said substrate to a product by said second hydrolase is inhibited by said inhibitor; and

(e) detecting said ligand by a signal associated with said product.

6. A method for the detection of an antigen in a liquid comprising:

(a) combining in an assay vessel a first liquid suspected of containing an antigen with first and second antibodies, said first antibody being conjugated to a magnetic particle and said second antibody having alkaline phosphatase conjugated thereto whereby said antigen binds to said first and second antibodies to give a bound phase on said particle;

(b) separating said particle having bound phase thereon from said first liquid;

(c) contacting the separated particle with a second liquid containing an esterase and a blocked fluoroketone inhibitor whereby said alkaline phosphatase converts said blocked fluoroketone inhibitor to a fluoroketone inhibitor of said esterase, said fluoroketone being released into said second liquid;

(d) adding to said second liquid a chromogen, the conversion of which to a colored product by said esterase is inhibited by said fluoroketone; and

(e) detecting said antigen by detecting inhibition of formation of color in said second liquid.

7. The method in accordance with Claim 6 wherein said esterase is conjugated to siad magnetic particle.

8. A kit of materials for performing an assay for a ligand comprising an antiligand conjugated to a magnetic particle, a first hydrolase conjugated to one of said ligand and a second antiligand, and a blocked inhibitor for a second hydrolase.

9. The kit in accordance with Claim 8 further comprising said second hydrolase.

10. The kit in accordance with Claim 8 further comprising at least one other reagent selected from the group consisting of an enzyme substrate, antigen, antibody and antibody complex, buffer and saline.

European Patent Office

# EUROPEAN SEARCH REPORT

Application Number

EP 90 10 4248

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 271 731 (BECTON, DICKINSON & CO.) * Pages 1-10 * | 1-10 | G 01 N 33/542<br>G 01 N 33/58<br>G 01 N 33/553 //<br>G 01 N 33/569 |
| Y | EP-A-0 149 565 (AMERSHAM INT. plc) * Pages 1-8 * & US-A-4 745 077 (Cat. D) | 1-10 | |
| Y | EP-A-0 245 981 (BECTON, DICKINSON & CO.) * Page 4, line 23 - page 6, line 10; page 9, line 1 - page 19, line 30 * | 1-10 | |
| Y | EP-A-0 240 770 (E.I. DU PONT DE NEMOURS & CO.) * Pages 1-2; page 15, line 3 - page 16, line 27; page 18, line 30 - page 20, line 30 * | 1-10 | |
| Y | BIOCHEMISTRY, vol. 24, no. 8, 9th April 1985, pages 1813-1817, American Chemical Society; M.H. GELB et al.: "Fluoro ketone inhibitors of hydrolytic enzymes" * Whole article * | 1-10 | TECHNICAL FIELDS SEARCHED (Int. Cl.5)<br>G 01 N |
| A | EP-A-0 027 036 (C.H. SELF) & US-A-4 446 231 (Cat. D) | | |

The present search report has been drawn up for all claims

| Place of search | Date of completion of the search | Examiner |
|---|---|---|
| THE HAGUE | 06-06-1990 | HITCHEN C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document